# EUROPEAN PATENT APPLICATION

(11) **EP 4 640 775 A1**
(43) Date of publication of application: **29.10.2025**
(21) Application number: 23906160.9
(22) Date of filing: 15.12.2023
(51) Int. Cl.: C09D 139/04, C09D 201/02, C08F 8/02, C08F 2/48, C08F 226/06, C08J 3/24, C08J 7/04, C12N 5/00

(54) **METHOD FOR PREPARING TRANSPARENT AND HEAT-SENSITIVE POLYCAPROLACTAM COATINGS ON POLYMER SUBSTRATES**

(30) Priority: 20.12.2022 ES 202231082
(71) Applicant: Consejo Superior De Investigaciones Científicas (CSIC), 28006 Madrid (ES)
(72) Inventor: REINECKE, Helmut, 28006 Madrid (ES); GALLARDO RUIZ, Alberto, 28006 Madrid (ES); RODRÍGUEZ HERNÁNDEZ, Juan, 28006 Madrid (ES); MARTÍNEZ CAMPOS, Enrique, 28006 Madrid (ES); ELVIRA PUJALTE, Carlos, 28006 Madrid (ES)
(74) Representative: Pons IP
(86) International application number: PCT/ES2023/070753
(87) International publication number: WO 2024/133996

(57) **Abstract**

The present invention relates to a method for preparing a transparent and heat-sensitive polyvinylcaprolactam (PVCL) coating photochemically attached to a polymer support or substrate. The method comprises: a) synthesising a copolymer having caprolactam and benzophenone groups; b) contacting the copolymer synthesised in step a) with the surface of a polymer to be modified; and c) exposing the surface to be modified, contacted with the polymer synthesised in step a), to UV light.

## Description

The present invention relates to a process for the preparation of a polyvinylcaprolactam (PVCL) coating photochemically anchored to a polymeric substrate or support. This invention allows for the production of thermosensitive and completely transparent coatings through the use of unprecedented synthetic strategies that allow the obtaining of activated polyvinylcaprolactam through compositionally homogeneous copolymerizations. Therefore, the present invention belongs to the intelligent and active surfaces sector.

### BACKGROUND TO THE INVENTION

The preparation of polymeric supports with a consistent PVCL coating is of biomedical interest since these water-swollen materials not only favour the growth, proliferation and formation of cell monolayers of different types at 37°C, but, thanks to their thermosensitivity, they allow easy and non-aggressive detachment and transplantation of the cells due to a drop in temperature. It is essential that this coating is stable/insoluble under the experimental conditions applied. In addition, it is desirable that the coating be transparent, so that the automatic reading of cells anchored to the support (which are stained so that a reader can measure their intensity) is allowed.

A procedure has recently been described (WO2018130739) to obtain polymeric substrates with a hydrogel-like coating based on N-vinylcaprolactam (VCL) where the coating has been anchored to the polymeric substrate. The preparatory method for creating this type of coating consists of 1) preparing a mixture of the hydrogel precursor monomers, which are the vinyl derivative caprolactam, at least two crosslinking agents, a photoinitiator and, optionally, a solvent, 2) deposition of the mixture on the surface of the polymeric substrate and 3) induction of photopolymerization under UV radiation. As a result of the diffusion of the hydrogel precursor molecules into the outer layers of the polymer substrate, an interface is formed to which the hydrogel coating is irreversibly anchored after photocuring. By immersion in water or ethanol of the coated support, the extraction of residual monomer that has not reacted and the swelling of the hydrogel is achieved.

An experimental problem with this methodology is the need for very strict control of its parameters; thus, if the thickness is too large, the substrate loses its transparency and wrinkles form on the surface of the hydrogel; In addition, it is problematic to find the optimal time and temperature of the deposition process on the support and curing since there is a strong tendency to crystallization of the mixture which prevents the obtaining of homogeneous surfaces.

Therefore, there is a need to find an alternative process to obtain supports with PVCL-based coatings that are thermosensitive and transparent for subsequent application in biomedicine.

### DESCRIPCION DE LA INVENCION

In the present invention, radical polymerization is applied to obtain and anchor PVCL to polymeric surfaces, preferably polystyrene, in order to obtain systems that facilitate the adhesion, proliferation and detachment in non-aggressive conditions of cell cultures and monolayers thereof. Since VCL is a low-activated monomer for radical polymerization, this invention describes a benzophenone (BP) activation strategy based on the use of other low-activated precursors to obtain copolymers with high compositional homogeneity, which have allowed transparent and thermosensitive coatings to be obtained.

Then, a first aspect of the invention refers to a process for the preparation of transparent and thermosensitive PVC coatings on polymeric substrates that comprises the following steps:
a) synthesising a copolymer carrying caprolactam and benzophenone groups, as shown below: 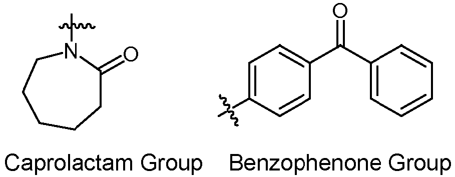 where this synthesis is carried out through one of the following synthetic routes:
   a.1) the synthesis of a copolymer from N-vinylcaprolactam (VCL) and a vinyl monomer selected from N-vinylformamide and 3-aminopropyl vinyl ether, followed by acid hydrolysis only in the case of the copolymer obtained from VCL and N-vinylformamide in order to hydrolyse the amide groups to amine (-NH₂), and subsequent reaction of benzophenone 4-isocyanate with the copolymer obtained from VCL and 3-aminopropyl vinyl ether or the copolymer obtained after the acid hydrolysis (this step will allow the anchoring of the BP to the copolymer),
   (a.2) the synthesis of a vinyl monomer incorporating benzophenone from 3-aminopropyl vinyl ether, or 2-hydroxyethyl vinyl ether, and benzophenone-4-isocyanate and subsequent copolymerization reaction of the formed vinyl monomer and VCL,
(b) contacting the copolymer synthesized in step (a) with the surface of the substrate whose surface is to be coated, and
(c) exposing to UV light of the substrate that has come into contact with the copolymer synthesized in step (a), thus allowing the copolymer to anchor to the surface of the substrate and, at the same time, the formation of a polymeric network which, when swollen in water, will form a hydrogel.

Optionally, the procedure may include a step d) of swelling the product obtained in c) by immersing it in water or ethanol, thus forming a hydrogel coating.

The monomers used in stage a) are especially relevant to obtain a clear final coating based on VCL. Many VCL-derived copolymers have significant compositional heterogeneity, especially those obtained with monomers that are more reactive than VCL and that polymerize more easily, since this different reactivity gives rise to copolymers with a compositional gradient that is macroscopically reflected in a turbidity of the polymer in solution and, as a consequence, in opaque coatings. In certain applications where full transparency of the coated surfaces is required, this turbidity is unacceptable and an alternative solution is required. This invention describes two synthetic strategies (a.1) and (a.2) to achieve compositionally homogeneous copolymers of VCL and benzophenone-carrying monomers that result in transparent coatings.

In a preferred embodiment, the polymer substrate is selected from polystyrene (PS), polymethyl methacrylate (PMMA), nylon, polycarbonate (PC), PVC (polyvinyl chloride), polylactic acid (PLA) or polycaprolactone (PCL). In a more preferred example, the polymer substrate is polystyrene.

In a preferred embodiment, the synthesis of a copolymer in step a) is carried out according to the synthetic route a.1) by copolymerization of VCL and N-vinylformamide or 3-aminopropyl vinyl ether (Schedule 1a and 2a). The copolymerization reaction of VCL and N-vinylformamide or 3-aminopropyl vinyl ether is a radical copolymerization that is preferably carried out in the presence of 2,2'-azobis(2-methylpropionitrile) (AIBN) as an initiator and/or at a preferred temperature between 50 and 70°C and/or, preferably, dioxane, DMF (dimethylformamide) or ethanol as a solvent. In a preferred embodiment, the amount of AIBN to VCL is between 0.01 and 0.02, more preferably, 0.015 mol AIBN per mol VCL. In another preferred embodiment, the amount of N-vinylformamide or 3-aminopropyl vinyl ether is between 1.0 and 5.0 mol% with respect to the VCL.

For the subsequent anchoring of benzophenone to the copolymer formed by VCL and N-vinylformamide, it is necessary that both this copolymer and benzophenone have functional groups that can react with each other in order to form a covalent bond. To do this, the copolymers obtained by copolymerization of VCL and N-vinylformamide are subjected to acid hydrolysis. This hydrolysis is carried out in water and hydrochloric acid in a preferred concentration between 1 and 3 molar, at a preferred temperature between 50 and 90°C, so that the amide groups present in the copolymer are transformed into primary amines (Diagram 1a).

Additionally, benzophenone functionalized with an isocyanate group (4-benzophenone isocyanate) is preferably obtained from 4-aminobenzophenone by treatment of the latter with triphosgene and triethylamine (Diagram 1b). In a preferred embodiment, 0.3 to 0.4 moles of triphosgene are used, plus preferably 0.33 moles and/or 2 to 3 moles of triethylamine, plus preferably, 2 moles for each mole of 4-aminobenzophenone.

Finally, the copolymer subjected to acid hydrolysis, or the copolymer obtained directly by copolymerization of VCL and 3-aminopropyl vinyl ether, is reacted with the amount of benzophenone-4-isocyanate equivalent to the moles of amine groups of the copolymer to give rise to the corresponding final copolymer carrying caprolactam and benzophenone groups (diagram 1c and 2b). The reaction is preferably carried out in absolute THF. In a preferred embodiment, the copolymer obtained directly by copolymerization of VCL and 3-aminopropyl vinyl ether is found at a concentration between 0.05 and 0.2 g/mL, plus preferably 0.1 g/mL in the solution for reaction with 4-benzophenone isocyanate.

In a preferred embodiment, the copolymer thus formed is purified by simple precipitation in diethylether.

The advantage of synthesizing the copolymer from VCL and 3-aminopropyl vinyl ether is that the hydrolysis step is not required to form an amine -NH₂ group capable of reacting with 4-benzophenone isocyanate, thus saving a synthetic step.

**Diagram 1:** Synthetic route a.1) from N-vinylformamide: a) preparation of VCL copolymer with N-vinylformamide and subsequent hydrolysis to primary amines; (b) transformation of 4-aminobenzophenone into the corresponding isocyanate; (c) anchoring by urea group formation of benzophenone units to the amino copolymer. 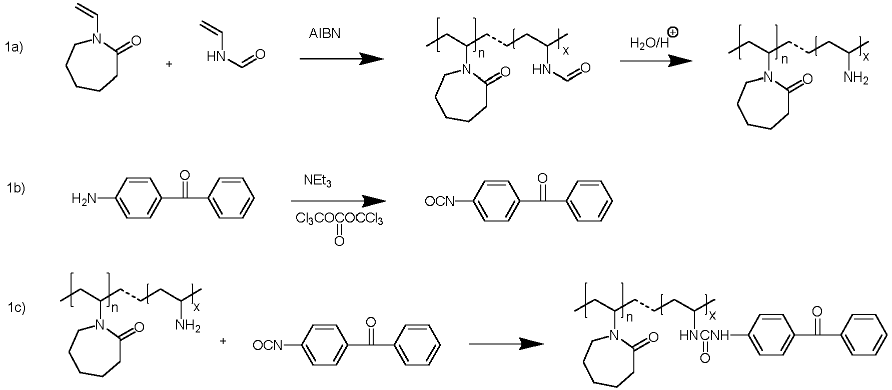 where, in a preferred realization, x/(n+x), which is the molar fraction of the amino component, is in the range 0.01< x/(n+x)< 0.05.

Diagram 2: Synthetic route a.1) from 3-aminopropyl vinyl ether: a) preparation of compositionally homogeneous copolymer between VCL and 3-amino vinyl ether and b) subsequent anchoring of benzophenone-4-isocyanate by urea bonds. 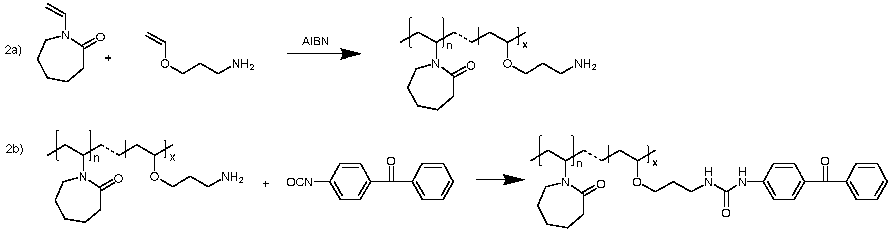 where, in a preferred realization, x/(n+x), which is the molar fraction of the amino component, is in the range 0.01< x/(n+x)< 0.05.

In another preferred embodiment, the synthesis of a copolymer in step (a) is carried out according to the synthetic route (a.2). This route avoids the possible formation of low molecular weight polymers (due to transfer reactions due to the amine groups present in the polymerization) and consists of preparing, in a first step, a vinyl monomer that already contains benzophenone. To do this, 3-aminopropyl vinyl ether, or 2-hydroxyethyl vinyl ether, is reacted with 4-benzophenone isocyanate. The product is obtained quantitatively and can be used for copolymerization with VCL (diagram 4). This copolymerization is also a classical radical copolymerization that is preferably carried out in the presence of 2,2'-azobis(2-methylpropionitrile) (AIBN) as an initiator and/or at a preferred temperature between 50 and 70°C and/or, preferably, dioxane, DMF (dimethylformamide) or ethanol as a solvent. In a preferred embodiment, the amount of AIBN with respect to the VCL is between 0.01 and 0.02 mol AIBN per mole VCL, more preferably, 0.015 mol AIBN per mole VCL. In another preferred embodiment, the amount of vinyl monomer already contained in benzophenone is between 1.0 and 5.0 mol % compared to the VCL.

The reactions of 3-aminopropyl vinyl ether, or 2-hydroxyethyl vinyl ether, with benzophenone-4-isocyanate are preferably carried out using the reagents in stoichiometric amounts. In a preferred embodiment, this reaction is carried out in a solution of dry THF or CH₂Cl₂ and/or at a preferred concentration between 0.05 and 0.2 g/ml and/or in a temperature range between 0 and 6°C. 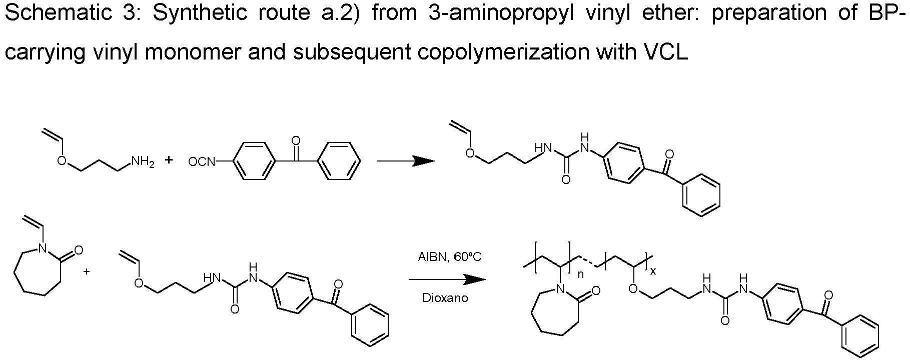 where, in a preferred realization, x/(n+x), which is the molar fraction of the amino component, is in the range 0.01< x/(n+x)< 0.05.

In a preferred embodiment, the copolymer obtained in step (a) is dissolved in a solvent, preferably in a concentration of between 3 and 25 mg of copolymer per ml of solvent, and applied by spraying, spin coating or dipcoating to the surface to be modified. Any liquid that dissolves the copolymer and does not interact with the substrate is used as a solvent. In a preferred production, alcohols with boiling points below 100°C are used. In a more preferred embodiment, ethanol is used as a solvent.

The thickness of the coating is adjusted using the appropriate concentration of the copolymer in the solvent. To create a coating thickness or thickness between 1 and 10 µm, a concentration of between 3 and 25 mg of copolymer per ml of solvent is used.

The dry coating is anchored to the substrate by exposing it to UV light, preferably in a light-curing chamber.

In a preferred embodiment, the wavelength of the UV light used in stage c) is 365 nm.

In another preferred embodiment, the UV light exposure time in stage c) is between 20 and 60 minutes.

Another aspect of the invention relates to a material comprising a polymeric substrate, preferably polystyrene, and a coating covalently bonded to the surface of such substrate, where such coating comprises a copolymer carrying caprolactam and benzophenone groups, where such material is obtained by the process described in the first aspect of the invention.

In a preferred embodiment, the polymer substrate is selected from polystyrene (PS), polymethyl methacrylate (PMMA), nylon, polycarbonate (PC), PVC (polyvinyl chloride), polylactic acid (PLA) or polycaprolactone (PCL). In a more preferred example, the polymer substrate is polystyrene.

The coating of the material is thermosensitive and transparent.

In a preferred realization, the coating thickness is between 1 and 50 µm, more preferably, between 1 and 10 µm.

The present invention also relates to copolymers obtained by means of synthetic routes a.1) and a.2) indicated above and whose formulas are as follows: 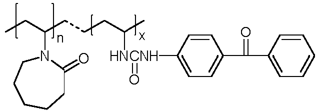 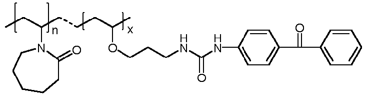 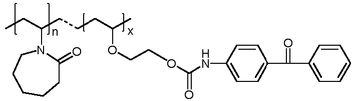 where, in a preferred realization, x/(n+x), which is the molar fraction of the amino component, is in the range 0.01< x/(n+x)< 0.05.

Throughout the description and claims, the word "comprises" and its variants are not intended to exclude other technical characteristics, additives, components or steps. For experts in the field, other objects, advantages and characteristics of the invention will follow partly from the description and partly from the practice of the invention. The following examples and figures are provided by way of illustration, and are not intended to be limiting to the present invention.

### BRIEF DESCRIPTION OF THE FIGURES

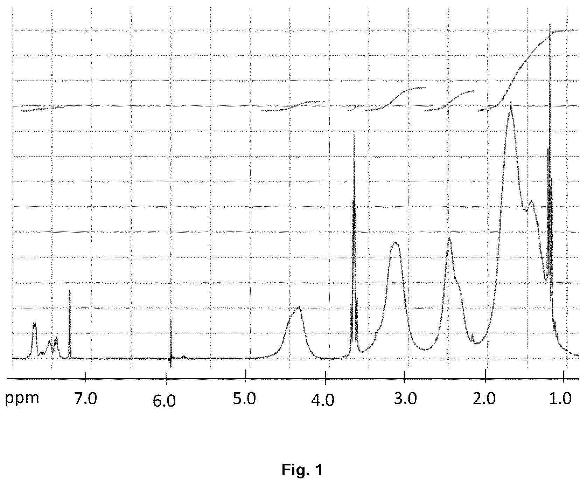

**Fig. 1****:** ¹H-NMR of the copolymer obtained in example 3.

### EXAMPLES

Next, the invention will be illustrated by some examples carried out by the inventors.

### Example 1: Preparation of compositionally homogeneous copolymers according to route a.1) from VCL and vinylformamide

Dissolve 9 g (96.7 mol%) of VCL and 157 mg (3.3 mol%) of vinylformamide in 67 ml of dioxane, add 165 mg of AIBN and degas the mixture by passing a flow of N₂ through the solution for 30 minutes. It is then heated for 24 hours at 60°C. The dioxane is removed and 60 ml H₂O and 5 ml hydrochloric are added to the formed copolymer and heated for 20 hours at 70°C. Subsequently, it is dialyzed in dialysis bags with an exclusion size of 1000 and the copolymer is freeze-dried.

Additionally, 4-aminobenzophenone is transformed by triphosgene and triethylamine into the corresponding isocyanate. To do this, 219 mg triphosgene are dissolved in 30 ml dichloromethane and, on the other, 436 mg 4-amino benzophenone together with 0.65 ml triethylamine in 50 ml dichloromethane. The solution of the amines is added to the triphosgene solution and the mixture obtained is used for the next step.

Finally, the copolymer of the aminated VCL is dissolved in 50 mL CH₂Cl₂ and mixed with the isocyanate of benzophenone. The dichloromethane is removed after 5 minutes, the residue is dissolved in ethanol and the solution is purified by dialysis in water (dialysis bag of 1000). After freeze-drying the contents of the dialysis bags, 9.4 g of a VCL-based copolymer containing 4 mol% benzophenone units is obtained and used to create coatings on polystyrene plates.

### Example 2: Preparation of compositionally homogeneous copolymers according to route a.1) from VCL and 3-aminopropyl vinyl ether

Dissolve 9 g (95.0 mol%) of VCL and 344 mg (5.0 mol%) of 3-aminopropyl vinylether in 68 ml of dioxane, add 168 mg of AIBN and degas the mixture by passing a flow of N₂ through the solution for 30 minutes. It is then heated for 24 hours at 60°C. Dioxane is removed and replaced by the same amount of CH₂Cl₂.

Additionally, 4-aminobenzophenone is transformed by triphosgene and triethylamine into the corresponding isocyanate (Diagram 1b). To do this, 337 mg triphosgene is dissolved in 30 ml dichloromethane and, on the other, 672 mg 4-amino benzophenone together with 1.0 ml triethylamine in 50 ml dichloromethane. The solution of the amines is added to the triphosgene solution and the mixture obtained is used for the next step.

Finally, the copolymer of the amination caprolactam (Schedule 2a) is dissolved in 60 mL CH₂Cl₂ and mixed with the isocyanate of benzophenone (Schedule 2b). The dichloromethane is removed after 5 minutes, the residue is dissolved in ethanol and the solution is purified by dialysis in ethanol (1000 dialysis bag). After removal of ethanol, 10.6 g of a caprolactam-based copolymer carrying 4 mol% benzophenone units is obtained and used to create coatings on polystyrene plates.

### Example 3: Preparation of compositionally homogeneous copolymers according to route a.2)

First, the monomer is prepared by reaction of benzophenone-4-isocyanate and 3-aminopropyl vinyl ether. Dissolve 5 grams of benzophenone-4-isocyanate in 50 mL of CH₂Cl₂ and add, at room temperature, a solution of 2.26 grams of 3-aminopropyl vinyl ether dissolved in 50 mL of CH₂Cl₂. Dichloromethane is removed and the residue is suspended in THF. The suspension is filtered and THF is removed from the filtrate. The product obtained is of sufficient purity to be used for copolymerisation with VCL. Dissolve 9 g (95 mol%) of VCL and 1.13 g (5.0 mol%) of the monomer obtained by reaction of 4-benzophenone isocyanate and 3-aminopropyl vinyl ether in 68 ml of dioxane, add 168 mg of AIBN and degas the mixture by passing a flow of N₂ through the solution for 30 minutes. It is then heated for 24 hours at 60°C. The copolymer obtained in diethylether is precipitated.

### Example 4: Preparation of PVCL-coated polystyrene (PS) sheets

A solution of between 20 and 50 mg of the copolymer obtained in any of the 1 to 3 examples is prepared in 1 ml of ethanol. The solution is filtered so that it is free of dust. Each of the 12 wells of a PS plate (diameter 2 cm each) is filled with between 150 and 200 µL of the solution. The solvent evaporates at room temperature and puts the plate in the curing chamber and illuminates with UV light (365 nm) for 40 minutes. In all cases, transparent coatings were obtained with thicknesses that vary according to the amount of copolymer and the volume used to fill the wells. Typical thickness values of between 5 and 50 microns are obtained.

## Claims

1. Procedure for the preparation of transparent and thermosensitive polyvinyl-caprolactam coatings on polymeric substrates comprising the following steps:
a) synthesizing a polyvinyl-caprolactam copolymer containing caprolactam and benzophenone groups, 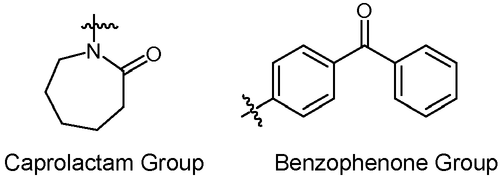 where this synthesis is carried out through one of the following synthetic routes:
(a.1) the synthesis of the copolymer from N-vinylcaprolactam (VCL) and a vinyl monomer selected from N-vinylformamide and 3-aminopropyl vinyl ether, followed by acid hydrolysis only in the case of the copolymer obtained from VCL and N-vinylformamide in order to hydrolyse the amide groups to amine, and subsequent reaction of benzophenone 4-isocyanate with the copolymer obtained from VCL and 3-aminopropyl vinyl ether or that obtained after acid hydrolysis, or
(a.2) synthesis of a vinyl monomer incorporating benzophenone from 3-aminopropyl vinyl ether, or 2-hydroxyethyl vinyl ether, and isocyanate from 4-benzophenone and subsequent copolymerization reaction of the formed vinyl monomer and VCL,
(b) contacting the copolymer synthesized in step (a) with the surface of the substrate whose surface is to be coated, and
(c) exposing to UV light of the substrate that has come into contact with the copolymer.

2. Procedure, according to claim 1, wherein the polymeric substrate is polystyrene.

3. Procedure, according to claim 1 or 2, wherein the synthesis of the copolymer in step a) is carried out according to the synthetic route a.1) by copolymerization of VCL and N-vinylformamide or 3-aminopropyl vinyl ether, wherein such copolymerization is carried out in the presence of 2,2'-azobis(2-methylpropionitrile) (AIBN) as initiator, at a temperature between 50 and 70°C and/or in dioxane, dimethylformamide or ethanol as solvent.

4. Procedure, according to any of the previous claims, wherein the synthesis of the copolymer in step (a) is carried out according to the synthetic route a.1) where the amount of N-vinylformamide or 3-aminopropyl vinyl ether used is between 1.0 and 5.0 mol % with respect to the VCL.

5. Procedure, according to any of the previous claims, wherein acid hydrolysis of the VCL-N-vinylformamide copolymer is carried out in water and hydrochloric acid in a concentration between 1 and 3 molar and/or at a temperature between 50 and 90°C, thus forming an amino copolymer.

6. Procedure, according to claim 4, where, following the synthetic route a.1), the copolymer subjected to acid hydrolysis, or the copolymer obtained directly by copolymerization of VCL and 3-aminopropyl vinyl ether, is reacted with the amount of benzophenone-4-isocyanate equivalent to the moles of amine groups of the copolymer to give rise to the final copolymer.

7. Procedure, according to claim 1 or 2, wherein the synthesis of the copolymer in step a) is carried out according to the synthetic route a.2) by reaction of 3-aminopropyl vinyl ether or 2-hydroxyethyl vinyl ether with benzophenone 4-isocyanate wherein the reagents are used in stoichiometric quantities and/or at temperatures between 0 and 6°C

8. Procedure, according to claim 1, 2 or 7, wherein the copolymerization reaction of the obtained vinyl monomer and the VCL according to synthetic route a.2) is carried out in the presence of 2,2'-azobis(2-methylpropionitrile) (AIBN) as initiator, at a temperature between 50 and 70°C and/or dioxane, dimethylformamide or ethanol as solvent.

9. Procedure, according to claim 1, 2, 7 or 8, wherein the amount of vinyl monomer containing the benzophenone is between 1.0 and 5.0 mol % with respect to the VCL in the copolymerization reaction in the synthetic route a.2).

10. Procedure, according to any of the previous claims, wherein the copolymer obtained in step a) is dissolved in a solvent for contacting the substrate surface.

11. Procedure, according to claim 10, wherein the copolymer is applied to the surface of the substrate by spraying, rotational coating or immersion coating.

12. Procedure, according to any of claims 9 or 10, wherein the solvent in step b) is an alcohol with a boiling point below 100°C.

13. Process, according to any of the previous claims 9 to 11, wherein the solvent in step b) is ethanol.

14. Procedure, according to any of the previous claims, wherein the exposure of step c) is carried out at 365 nm.

15. Procedure, according to any of the previous claims, wherein the exposition step c) is carried out for a period of between 20 and 60 min.

16. Procedure, according to any of the previous claims, which includes an additional step (d) of swelling of the product obtained in step (c) by immersion in water or ethanol, thereby forming a hydrogel coating.

17. Material comprising a polymeric substrate and a coating covalently bonded to the surface of such substrate, where such coating comprises a polyvinyl-caprolactam copolymer containing caprolactam and benzophenone groups, wherein such material has been obtained by the process described in any of claims 1 to 16.

18. Copolymer of formula selected from the following: 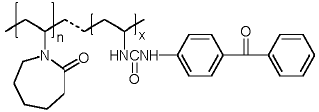 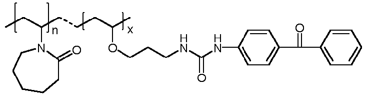 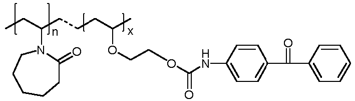 where, x/(n+x) is in the range: 0.01< x/(n+x)< 0.05.
